# EUROPEAN PATENT APPLICATION

(11) **EP 1 688 413 A1**
(43) Date of publication of application: **09.08.2006**
(21) Application number: 05002296.1
(22) Date of filing: 03.02.2005
(51) Int. Cl.: C07D 263/57, A61K 31/423, A61P 29/00, A61P 1/00

(54) **Benzoxazole derivatives for the prophylaxis and treatment of inflammatory bowel diseases**

(71) Applicant: Hikma Pharmaceuticals Co. Ltd., 11118 Amman (JO)
(72) Inventor: Jilani, Jamal, Dr., 11195 Amman (JO)
(74) Representative: Winkler, Andreas Fritz Ernst

(57) **Abstract**

The present invention relates to novel compounds for the prophylaxis and treatment of inflammatory bowel disease (IBD) via the administration of an effective amount in a suitable pharmaceutical dosage of agents that are active by themselves or can deliver tin the large intestine active forms of the drugs such as 5ASA or benzoxazole acetic acid or platelet activating factors. The mechanism of the release is based on bacterial cleavage of an azo linkage in the mammalian lower bowel to release the active compound(s).

## Description

### BACKGROUND OF THE INVENTION

The present invention provides novel compounds for the treatment of intestinal diseases, in particular Inflammatory Bowel Disease (IBD). In particular it provides a group of compounds according to formulae 1-3. These compounds are either active by themselves or they can deliver active species in the targeted tissue, here, large intestine.

IBD is a chronic, nonspecific, inflammatory and ulcerative diseases of the small intestine and/or colon, and may be characterized by bloody diarrhea. An example is ulcerative colitis. In ulcerative colitis the disease begins in the rectosigmoid area and may extend proximally, eventually involving the entire colon, or it may involve the large bowel all at once. On the other hand, Crohn's disease is an inflammatory disease of the GI tract that predominates in the small intestine and the large intestine. See Cecil, Textbook of Medicine, 1568-1578.

Treatment of IBD has been accomplished by several pharmaceutical compounds. Notably, adrenocorticosteroids, 5-aminosalicylic acid (5ASA) and other 5ASA derivatives, such as sulfasalazine are in current use. The adrenocorticosteroids may mask symptoms of intestine perforation and peritonitis and are generally only used for short term therapy (Goodman and Gilman 4th Ed. pg. 1634 (1970)), and major complications may occur despite corticosteroid therapy. Several attempts were done to deliver 5ASA to the required site of action (GI tract) with minimal absorption. These attempts include chemical modifications and physical formulation on 5ASA.

Sulfasalazine (SS) (2-Hydroxy-5-[4-(2-pyridylsulfamoyl)phenylazo]benzoic acid) represents one of the currently used drugs for IBD. SS is a pro-drug, that is, upon administration, biological processes act upon SS to produce the drug which has the desired biological activity. Upon oral administration, about one-third of a given dose of SS is absorbed from the small intestine. The remaining two-thirds is split in the colon by azo-reductase from bacterial flora into sulfapyridine (SP), and 5-aminosalicylic acid (5ASA) (Physican's Desk Reference 31st Ed. pg. 1250 (1977). See also Klotz, New Eng J. of Med 303, 1499 (1980)). It has been determined that the activity of SS comes from the 5ASA produced. SS is effective as a pro-drug because its relative insolubility prevents its complete absorption in the small intestine thus allowing delivery of SS to the site of action, i.e., the large intestine. Given separately, both SP and 5ASA are almost completely absorbed from the small intestine. While effective, SS has several severe side effects including blood dyscrasias and hypersensitivity reactions. This toxicity of SS is due almost entirely to the SP produced.

U.S. 4,412,992 and U.S. 5,498,608 disclose a group of 2-hydroxy-5-phenylazobenzoic acid derivatives (e.g. balsalazide disodium) for the treatment of IBD and colorectal cancer. Balsalzide deliver 5-ASA by action of azo-reductase bacteria which exist in the large intestine. U.S. 20040121967 A1 discloses novel compounds having 5ASA derivatives incorporating a sugar residue or a poly(ethylene glycol) chain for easier delivery of 5ASA to the gastrointestinal tract. US 0191186A1 represents another example of 5ASA chemical modifications to deliver 5ASA and immunomodulator after azobond cleavage.

Related to the subject of inflammation, U.S. 3,912,758 discloses a number of benzoxazole derivatives with systemic anti-inflammatory activity. Among these benzoxazoles, 4-fluorophenyl-alpha-methylbenzoxazole-5-acetic acid was marketed under the name of (Flunoxaprofen). Research reports revealed that this compound may act as lipooxygenase /cyclooxygenase inhibitor.

### OBJECT OF THE INVENTION

The object of the present invention is to provide compounds for the prophylaxis and treatment of intestinal diseases, in particular Inflammatory Bowel Disease (IBD), such as Crohn's disease, ulcerative colitis, travellers' diarrhea and colorectal cancer.

In particular, it is an object of the present invention to provide for the administration of an effective amount of a suitable pharmaceutical dosage of an agent which is either active by itself or can deliver active drugs, such as 5ASA or benzoxazole derivatives, to the large intestine.

### SUMMARY OF THE INVENTION

The present invention is related to a compound of formula (1) 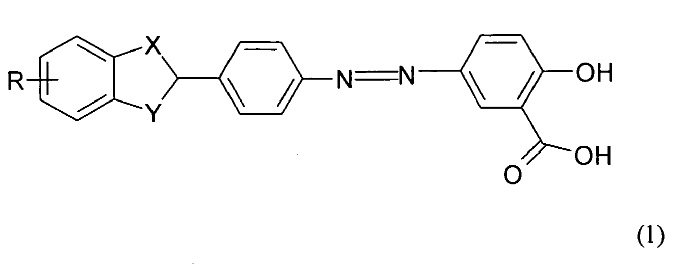 wherein X and Y are independently nitrogen, sulphur, oxygen or carbon; and R is 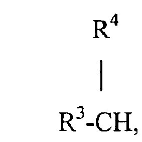 wherein R³ is selected from the group consisting of COOH or an ester or amide thereof or a substituted or unsubstituted tetrazole ring and wherein R⁴ is hydrogen or C₁-C₆ alkyl, or a pharmacologically acceptable salt or solvate thereof.

Moreover, the invention is related to a compound of formula (2) 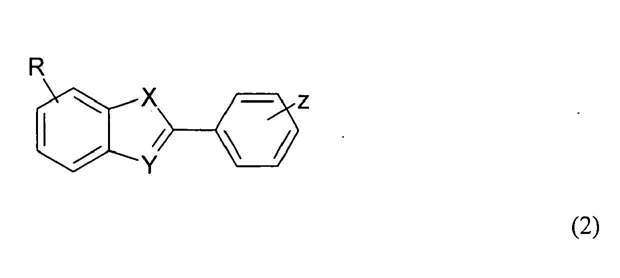 wherein R, X and Y are defined as in claim 1, and wherein Z is N=N-Phenyl-X²-NHR⁵, wherein X² is SO₂ or CO and R⁵ is an unsubstituted or substituted non-heterocyclic aromatic ring or an alkyl chain (CH₂)ₙM, wherein M is hydroxyl, amine, COOH or an ester or amide thereof or a salt-forming metallic cation, and n is an integer of from 1 to 6, or a pharmaceutically acceptable salt or solvate thereof.

Moreover, the invention is related to the use compounds of formula 2 in which R, X and Y are defined as above and in which Z is a substituted or unsubstituted amine, hydroxyl, halogen, a carboxylic acid, an ester of a carboxylic acid, an amide, or a substituted and unsubstituted tetrazole ring, or of a pharmaceutically acceptable salt or solvate thereof, for the preparation of a medicament for the treatment of an intestinal disease.

Furthermore, the invention is related to a compound of formula (3) 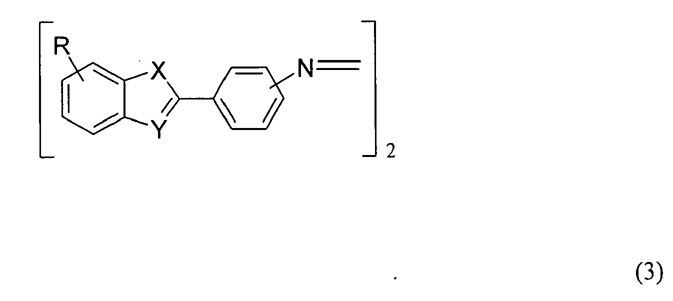 wherein X, Y and R are defined as hereinabove, or a pharmaceutically acceptable salt or solvate thereof.

The present invention is also related to pharmaceutical compositions comprising an effective amount of the respective compound, salt or solvate in admixture with a solid or liquid pharmaceutical diluent or carrier.

Furthermore, the invention is directed to the use of a compound, salt or solvate as defined hereinabove for the preparation of a medicament for the treatment of an intestinal disease. The intestinal disease is preferably one of the group consisting of Inflammatory Bowel Disease (IBD), Crohn's disease, ulcerative colitis, traveller's diarrhea and colorectal cancer.

The invention is also directed to a method for the synthesis of compounds of the invention.

Without wishing to be bound to any theory, the inventor believes that the mechanism of activity of the diazo compounds is based on bacterial cleavage of the azo-bond in the mammalian lower bowel to release the active compound(s).

### DETAILLED DESCRIPTION OF THE INVENTION

The preparation of the target compounds can be divided into two parts in order to simplify the discussion: non-diazo compounds and diazo compounds.

The non-diazo compounds are known per se and some of them have been prepared using known chemistry. The anti-inflammatory activity for some of them has been reported in U.S. 3,912,748.

Fig. 1 represents the described non-diazo compounds. The phenyl benzoxazole skeleton can be prepared according to different methods known from literature.

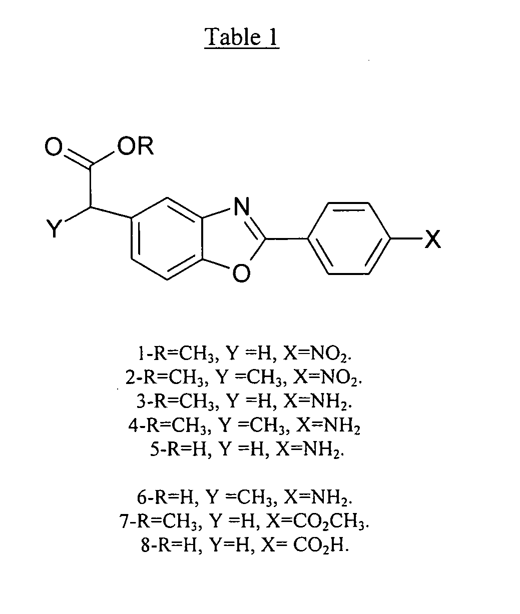

On the other hand, the diazo compounds are novel structures represented in Table 2.

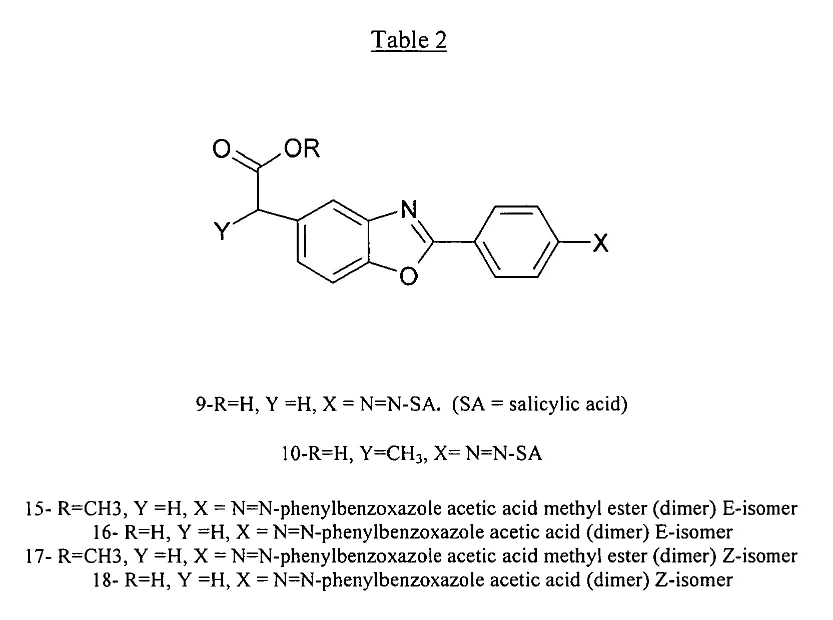

The preparation of the diazo compounds was attempted using different methods. In case of the salicylic acid diazo compounds (9 and 10), diazotization of the phenylbenzoxazole was done followed by the attack of salicylic acid (Scheme 1).

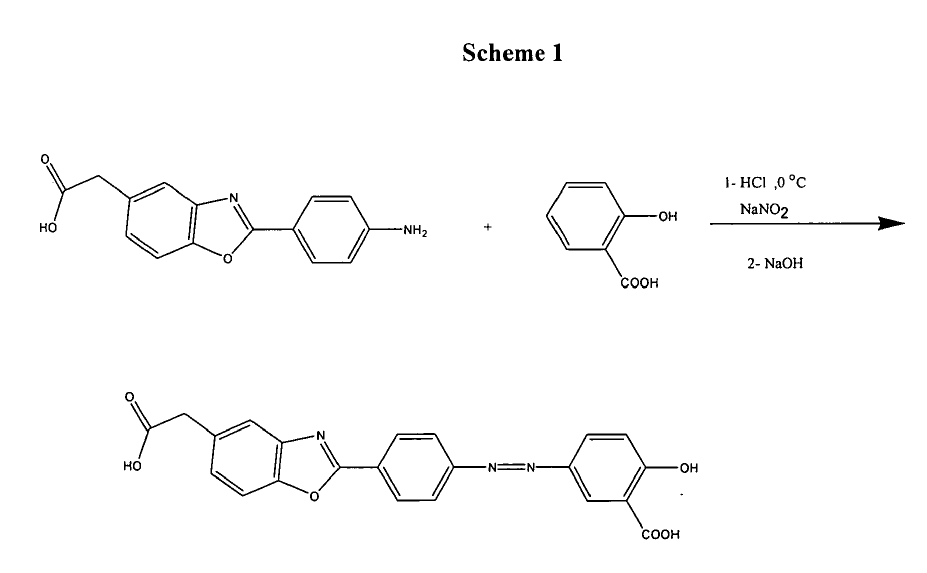

On the other hand, the symmetrical diazo dimers were prepared by reduction of the corresponding nitro compounds. Schemes 2 and 3 represent two synthetic routes that had been utilized to prepare the dimer diacids. In Scheme 2, the diazo bond was formed by the reduction of the nitro group of the protected 4-nitrobenzaldehyde using NaBH₄ or glucose to yield the corresponding diazo. On the other hand, the reduction of 4-nitrobenzoic acid using glucose gave a good yield of the diazo compound (Scheme 3). Although this reaction was reported (Tom-linson, M.L. J.Chem. Soc. 1946, pp756) and the corresponding diazo carboxylic acid and acid chloride are known, the regiochemistry of the products were not discussed. On the contrary, in this work the stereochemistry of the diazo were of concern. We found that glucose reduction of the 4-nitrobenzoic acid yielded mainly the relative E isomer, while the nitro reduction of the dioxolane yielded the relative Z isomer. These results may indicate the selectivity of the glucose reduction. The structure of the compound were proven by NMR, IR, and MS.

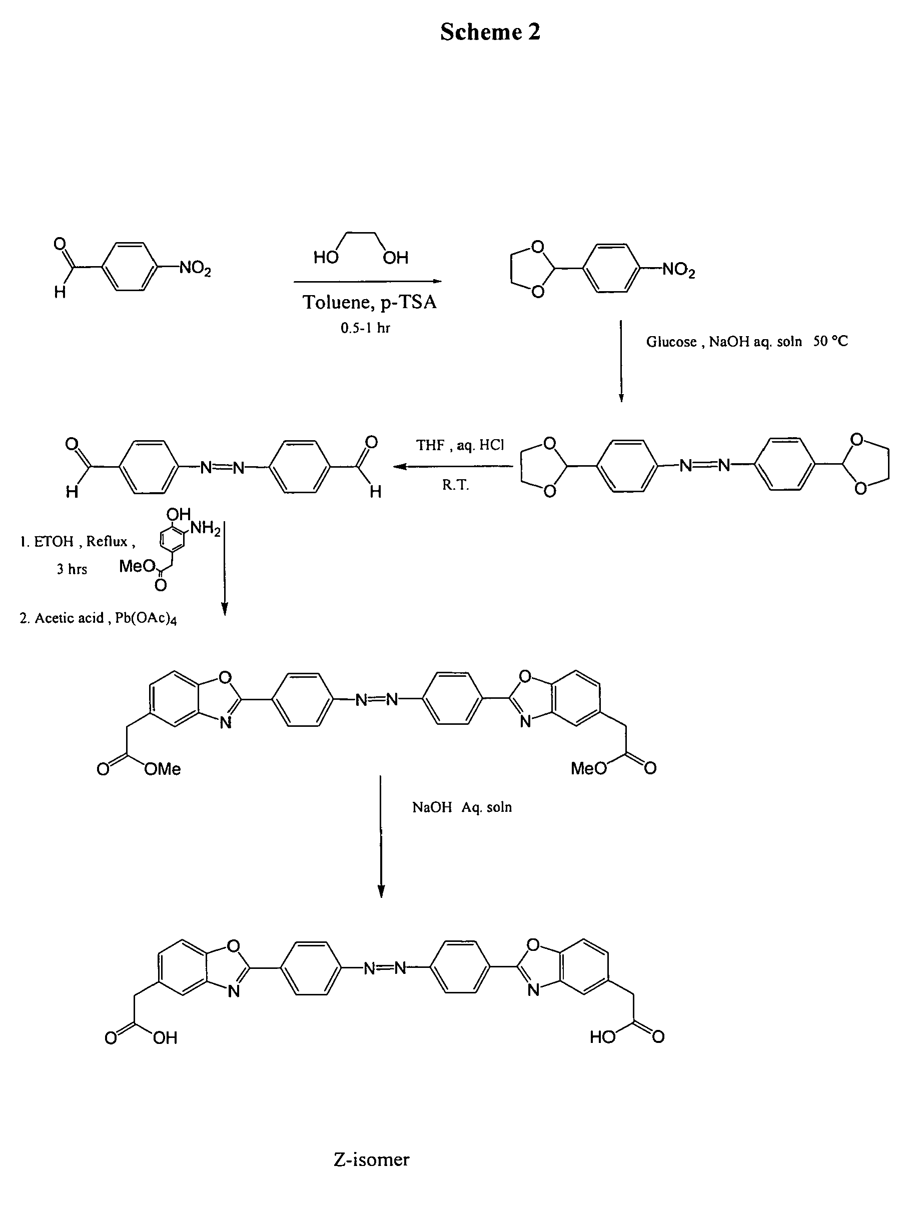

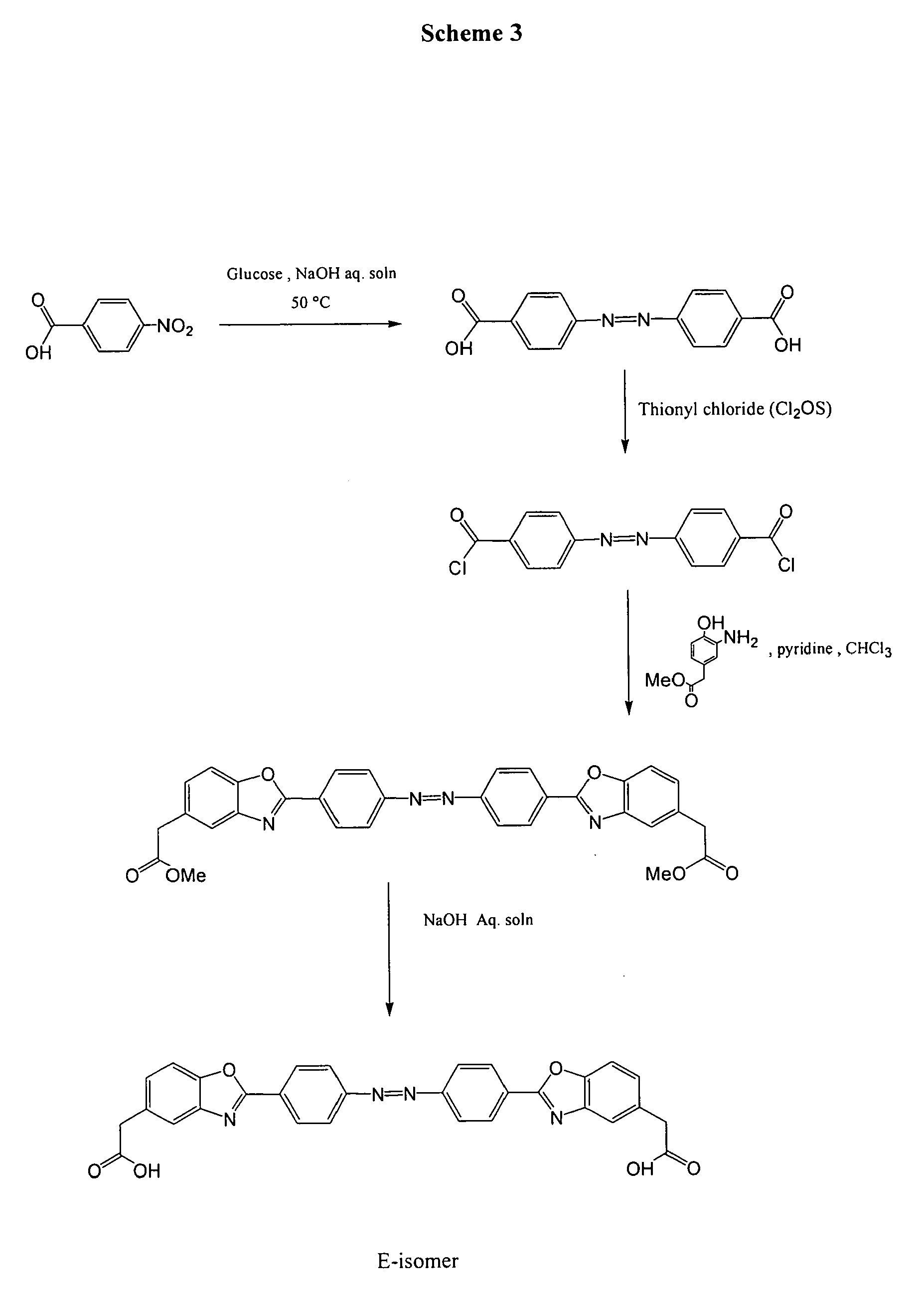

Melting points were taken on a Laboratory Devices Gallen Kamp model MPD 350.BM 2.5 capillary melting point apparatus and are uncorrected. ¹H NMR spectra were obtained on a Varian Unity 300 MHz spectrometer and chemical shifts (δ) are reported as parts per million (ppm) relative to internal standard tetramethylsilane. Infrared spectra were obtained with a Nicolet Impact 410. Mass spectroscopy data were obtained by (VG7070) M-Scan Inc. All reagents were used as received from commercially available sources.

### Example 1

### 2-Hydroxy-5-[4-(benzoxazol-2-yl-5-acetic acid)phenylazo]benzoic acid (9)

16.0 g of HCl salt of 4-aminophenylbenzoxazole-5-acetic acid 5 was suspended in 250 ml 6N HCl, cooled to 0°C. To this suspension, a pre-cooled (0°C) solution of sodium nitrite (5.0 g in 50 ml H₂O), was added dropwise within 15-20 minutes. The temperature of the mixture was kept between 0 ― 5°C. In an another flask 8.0 g (0.057 mole) of salicylic acid was dissolved in 200 ml of 25% solution of NaOH, cooled to 0°C. To this salt the solution of the diazonium salt was added drop wise within about 40-60 min while keeping the temperature at 0 ― 5°C and the pH above 8.0 (by adding NaOH solution). The mixture was stirred at 0 ― 5°C for 30 minutes and the pH was adjusted to about 7.0, then filtered. A red to orange colored powder was collected and washed with water. This powder was dissolved in 200 ml boiling 5% NaOH solution until a solution was formed, which was filtered, and the filtrate adjusted to a pH or 2.0. Upon cooling a precipitate was formed which was collected yielding 8.0 g of **9** as pure coloured solid.
m.p.: decomposition at 250 °C.
IR: (KBr) 1720 cm⁻¹.
¹H-NMR: *s(CH2), 3. 73; d (1H) 6.79 ; d (1H) 7. 34;* s *(1H) 7. 71; d (1H) 7.713; dd (1H) 7.86, d (2H), 7. 98; s (1H), 8.31; d (2H), 8.35.*

### Example 2

### 2-Hydroxy-5-[4-(benzoxazol-2-yl-5-alpha-methylacetic acid)phenylazolbenzoic acid (10) (rel. E isomer):

10 g (0.0338 mol) alpha-methyl-4-aminophenylbenzoxazole-5-acetic acid **6** was dissolved by heating in 200 ml of 6N HCl. The mixture was cooled to 0°C. A solution of sodium nitrite (3.3 g NaNO₂ in 75 ml H₂O) was added dropwise to the benzoxazole solution within 30 minutes while keeping the reaction temperature at 0°C.

In an another flask a solution of salicylic acid (5.4 g in 250 ml 20 % solution of sodium hydroxide) was prepared and cooled at 0°C.

To the salicylic acid solution, the diazonium salt solution was added dropwise within 30 minutes, keeping the pH of the mixture above 8.0 with the aid of sodium hydroxide solution. After the addition of the salt, stirring was continued at pH 8 for 30 min then adjusted to pH 3 at which a precipitation occurred. The mixture was cooled to 0-5°C , the powder collected by filtration, washed with water and dried. 5.6 g of crude ester was obtained and then dissolved in 30 ml solution sodium hydroxide with heating. The mixture was filtered to remove insolubles, then cooled down to about 20 °C and the pH adjusted to around 2.0. A solid precipitate was obtained, filtered and washed with water yielding 5.0 g of **10.**

### Example 3

### 2-(4-Nitrophenyl)-1,3-dioxolane (11)

p-Nitrobenzaldehyde (25 g, 0.165 mol) was dissolved in benzene (100 ml), and 3 crystals of p-toluene sulfonic acid, and ethylene glycol (10.5 g, 0.169 mol) were added. The mixture was refluxed for ½ hour. The benzene was distilled and freshly dried benzene was added to the reaction until no further water was collected with benzene. Ethylacetate (50 ml) was added and the solution was filtered. The filtrate was concentrated using a rotary evaporator to get 30-40 ml volume from which a crystalline solid was collected by filtration after cooling. The solid was washed with EtOAc and dried at 50 °C, yielding 23.8 g of 11 as pure solid. m.p. 88° - 92 °C.

### Example 4

### Bis (4-[1,3]-dioxolan-2-yl-phenyl) diazine (12).

To a mechanically stirred solution of sodium borohydride (5.4 g, 0.143 mol) in dimethylsulfoxide (40 ml), maintained at 85 °C, a solution of 2-(4-nitrophenyl)-1,3-dioxolane **11** (5 g, 0.0256 mol) in dimethylsulfoxide (19 ml) was added dropwise over 15 minutes. After 1.5 hour, water was added (240 ml), and extracted with chloroform (40ml x 4). The chloroformic extract was washed with 10% HC1, dried over anhydrous sodium sulfate and rotary evaporated yielding 4.34 g of solid. Purification with column gave 0.45 g, of **12.** mp 102-104 °C. IR (KBr) 1580 cm⁻¹.
MS fragmentation pattern:
326.3, 177, 149.2, 105.1, 73.1, 43.1, 28.0.

### Example 5

### 4,4'-Diazene-1,2-diylbenzaldehyde (12a).

0.45 g of diazo phenylacetal **(12)** was dissolved in tetrahydrofurane (25 ml) under N₂ atmosphere,10 ml HCl 10 % was added. After 2 hours of stirring the solution was evaporated yielding solid powder which was collected, washed with water, and dried giving 0.30 g of the diazo dialdehyde **12a**. mp 99-100 C.
MS: 28, 32, 75,105,119.2, 151.2, 179.2, 225.3, 253.3, 299.3, 330.
IR: (KBr) 1705 cm⁻¹.

### Example 6

### Azobenzene 4,4'-dicarboxylic acid (13)

4-Nitrobenzoic acid (5.2 g) and sodium hydroxide (20 g) were mixed with water (90 ml), the solution was warmed to 50°C, a solution of glucose (40 g) in 60 ml water was then added slowly at 50°C and the mixture was warmed in a steam bath, until a precipitate started to form.

Steam of air was drawn through the brown solution for 2 hours, until no further precipitate of the sodium salt of the product was formed on acidification (acetic acid). The mixture deposited azobenzene 4,4'-dicarboxylic acid which was collected by filtration yielding 3.5 g of **13,** m.p. > 300 C °.

### Example 7

### Azobenzene 4,4'-dicarboxylic acid chloride (14)

4 g of the azobenzene-4,4'-dicarboxylic acid **13** prepared above was refluxed with thionylchloride (60 ml) for 24 hours, and the precipitate was obtained by dilution with light petroleum, and recrystallized from light petroleum. The product **14** separated as red needles. Yield = 3.9 g, m.p. 164°C.

### Example 8

### 1,2-bis-[4-(5-methoxycarbonylmethylbenzoxazol-2-yl)phenyl]diazine (15) (rel. E isomer).

Azobenzene 4,4'-dicarboxylchloride **14** (0.36 g, 1.17 mmol) was dissolved in chloroform (10 ml), a solution of aminophenol (0.45 g, 2.48 mmol) in pyridine (2 ml) was added, stirred in a steam bath for one hour, the solid heated to 230 °C for 10 minutes. The mixture was dissolved in 3 ml DMF, filtered, ice/water was added and the precipitate formed was filtered and dried yielding 0.27g of **15.** m.p. 275°C . IR: C=O stretching at 1724 cm⁻¹. MS : 51, 77, 105, 122, 181, 223, 282, 398. ¹H NMR (DMSO d₆) ppm 3.2(s, 4H), 3.7 (d, 6H), 7.0 (m, 4H), 7.6(s, 2H), 8.0 (d, 4H), 8.2 (d,4H).

### Example 9

### 1.2-bis[4-(5-carboxymethylbenzoxazol-2-yl]phenyl]diazine (16). (rel E isomer)

To a solution of dicarboxylic acid chloride **14** (1.5 gm, 4.9 mmol) in 60 ml CHCl₃ was added a solution of aminophenol (1.77 g, 9.8 mmol) in 1.13 g pyridine. The mixture was heated in a water bath for 1 h, then heated for 10 minutes at 230°C. The resulting material was dissolved in DMF and addition of ice water formed a precipitate, which was redissolved in 5% NaOH solution. Acidification of the solution formed a precipitate which was collected and dried, yielding 1.24 g of the diacid **16.** m.p.>300°C. IR carbonyl 1690 cm⁻¹. MS: 44, 51, 63, 78, 92, 120, 137, 167, 223, 268.

### Example 10

### 1,2-bis-[4-(5-methoxycarbonylmethylbenzoxazol-2-yl)phenyl]diazine (17) (rel. Z isomer).

4,4'-Azobenzene dialdehyde **12** (0.55 g, 2.3 mmol) was dissolved in 28 ml ethanol, methyl-3-amino-4-hydroxyphenylacetate (0.84 g, 4.6 mol) was added. Reflux for 6 hours. Evaporate to dryness by rota evaporator. Hot acetic acid 35 ml was added then and while the reaction was warm lead tetraacetate (2.68 g, 6.0 mol) was added. The mixture was kept under stirring for 30 minutes, a brown precipitate formed, collected by filtration, washed with acetic acid and dried yielding 0.74 g of crude product which was purified by column yielding 0.345 g of **17.** m.p. = 285 °C. IR: (KBr) 1735 cm⁻¹
MS fragment pattern: 133, 207, 266, 282, 340, 399, 501, 560, 618

### Example 11

### 1,2-bis[4-(5-carboxymethylbenzoxazol-2-yl)phenyl]diazine (18) (rel Z isomer)

To a 100 mg of the diester **17** 5 ml of 10% NaOH was added, and refluxed for 30 min. Acidification with 6N HCl (8 ml) yielded a product which was filtered and then dissolved in hot DMF (5 ml). The solution was left to cool, then water was added yielding 33 g of the diacid **18** as solid powder. m.p. >300.

### Animal study:

Rats with TNB (trinitrobenzenesulfonic acid)-induced ulcerative colitis were used as an animal model.

28 rats were randomly grouped (A, B, C1, C2, D1 and D2). Group A is used as positive control and each of the other three groups received a predetermined dose of compounds under testing (see Table 3). Animals were fasted for one night before taking the TNB solution. All animals received a solution (0.25 ml) of TNB in methylcelullose solution (50% methylcellulose in absolute ethanol) containing 20 mg of TNB. The TNB solution was administered by intracolonic administration using a syringe and rubber canula. The canula was inserted into the lumen of the colon via the anus. The cannula was advanced so that its tip was 8 cm from the anus. After about 6 hrs after administering the TNB solution, 0.5 ml of compounds and placebo were given orally twice daily to each animal as follows:
1. Group A took 0.5 ml of the suspension containing 2.5mg of methylcellulose solution twice daily.
2. Group B took 0.5 ml of the suspension containing 5 mg of 5ASA twice daily.
3. Group C-1 took 0.5 ml of the suspension containing 18 mg of compound **9** twice daily.
4. Group C-2 took 0.5 ml of the suspension containing 28mg of compound **9** twice daily.
5. Group D-1 took 0.5 ml suspension containing 12 mg of compound **5** twice daily.
6. Group D-2 took 0.5 ml suspension containing 18mg of compound **5** twice daily.

The treatments were given for eight days , and on the 9^{th} day the animals were sacrificed and opened along their abdomen for evaluation.

The animals were subjected to the following testing
1. Physical testing , as looking at the weight, diahrrea, general health, etc.
2. Looking at the intestine before opening, colouration, thickness, etc.
3. Dissecting the intestine the colon and the cecum and examining them macroscopically and microscopically.

**Table 3**

| Rat No. | Mucosal inflammation and ulceration | Submucosal inflammation | Full thickness inflammation | Overall degree of inflammation |
|---|---|---|---|---|
| A1 | + | + | + + | + 2 |
| A2 | + + | + + | + + + | + 3 |
| A3 | + + | + + | + + + | + 3 |
| A4 | + | + | + | + 1 |
| A5 | + + | + + | + + | + 2 |
| | | | | |
| B1 | + + | + + | + + | + 2 |
| B2 | + + | + + | + + + | + 3 |
| B3 | + | + | + | + 1 |
| B4 | + + | + + | + + | + 2 |
| B5 | + + | + + | + + | + 2 |
| B6 | + + | + + | + + | + 2 |
| | | | | |
| C1-1 | - | - | - | - |
| C1-2 | + | ± | - | ± |
| C1-3 | + | - | - | ± |
| C1-4 | + | - | - | ± |
| C1-5 | + | + | - | + 1 |
| C1-6 | + | + | - | + 1 |
| C2-7 | + | ± | - | ± |
| C2-8 | + | ± | - | ± |
| C2-9 | + | + | - | + 1 |
| | | | | |
| D1-1 | + | + | + | + 1 |
| D1-2 | + | - | - | ± |
| D1-3 | + | + | + | + 1 |
| D1-4 | - | - | - | - |
| D1-5 | + | + | + | + 1 |
| D2-7 | + | + | - | + 1 |
| D2-8 | + | + | - | + 1 |
| D2-9 | + | + | - | + 1 |

| | | | | |
|---|---|---|---|---|
| - = No inflammation (normal), ± = Very minimal mucosal inflammation, | | | | |
| + = Mild, + 2= Moderate, + 3 = Severe | | | | |

The results show that both compounds **5** and **9** show significantly higher activity against intestinal bowel disease, compared to the known compound 5ASA. However, the absorption of the diazo compound is much less than that of the non-diazo compound. Therefore, use of the diazo compound for the treatment of intestinal diseases is safer and preferred, compared to the use of the known azo compounds such as compound **5.**

The features disclosed in the foregoing description and/or in the claims may, both separately and in any combination thereof, be material for realising the invention in diverse forms thereof.

## Claims

1. A compound of formula (1) 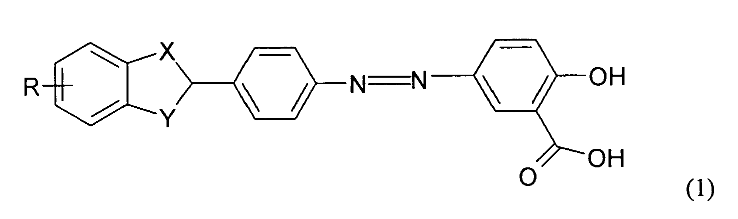 wherein X and Y are independently nitrogen, sulphur, oxygen or carbon; and R is 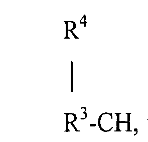 wherein R³ is selected from the group consisting of COOH or an ester or amide thereof or a substituted or unsubstituted tetrazole ring and wherein R⁴ is hydrogen or C₁-C₆ alkyl, or a pharmacologically acceptable salt or solvate thereof.

2. A pharmaceutical composition comprising an effective amount of a compound, salt or solvate according to claim 1 in admixture with a solid or liquid pharmaceutical diluent or carrier.

3. Use of a compound, salt or solvate according to claim 1 for the preparation of a medicament for the treatment of an intestinal disease.

4. A compound of formula (2) 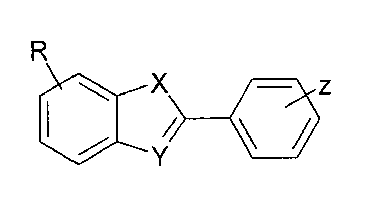 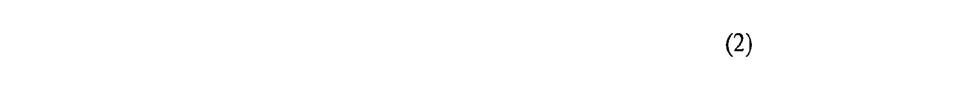 wherein R, X and Y are defined as in claim 1, and wherein Z is N=N-Phenyl-X²⁻NHR⁵, wherein X² is SO₂ or CO and R⁵ is an unsubstituted or substituted non-heterocyclic aromatic ring or an alkyl chain (CH₂)ₙM, wherein M is hydroxyl, amine, COOH or an ester or amide thereof or a salt-forming metallic cation, and n is an integer of from 1 to 6, or a pharmaceutically acceptable salt or solvate thereof.

5. A pharmaceutical composition comprising an effective amount of a compound, salt or solvate according to claim 4 in admixture with a solid or liquid pharmaceutical diluent or carrier.

6. Use of a compound, salt or solvate according to claim 4 for the preparation of a medicament for the treatment of an intestinal disease.

7. Use of a compound according to formula 4, wherein R, X and Y are defined as in claim 1 and wherein Z is a substituted or unsubstituted amine, hydroxyl, halogen, a carboxylic acid, an ester of a carboxylic acid, an amide, or a substituted and unsubstituted tetrazole ring, or of a pharmaceutically acceptable salt or solvate thereof, for the preparation of a medicament for the treatment of an intestinal disease.

8. A compound of formula (3) 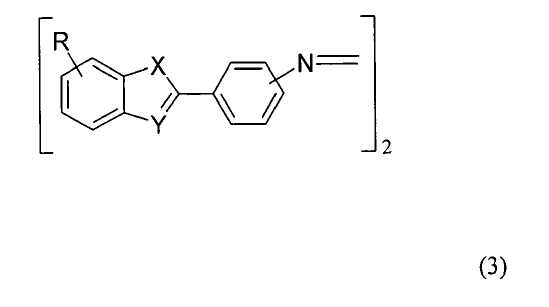 wherein X, Y and R are defined as in claim 1, or a pharmaceutically acceptable salt or solvate thereof.

9. A pharmaceutical composition comprising an effective amount of a compound, salt or solvate according to claim 8 in admixture with a solid or liquid pharmaceutical diluent or carrier.

10. Use of a compound, salt or solvate according to claim 8 for the preparation of a medicament for the treatment of an intestinal disease.

11. Use according to any of claims 3, 6, 7, or 10 wherein the intestinal disease is one of the group consisting of Inflammatory Bowel Disease (IBD), Crohn's Disease, ulcerative colitis, traveler's diarrhea and colorectal cancer.

12. Method for the synthesis of compounds according to formula 1, wherein the diazonium salt of an appropriate phenylbenzoxazole acetate derivative is prepared and then reacted with salicylic acid.

13. Method for the synthesis of compounds according to claim 8, wherein 4,4'-azobenzenedialdehyde is reacted with the appropriate aminophenyl derivative to obtain the diazo dimer.

14. Method for the synthesis of compounds according to claim 8, wherein 4,4'-azobenzenediacidchloride is reacted with the suitable aminophenol derivative to obtain the diazo dimer.
